# EUROPÄISCHE PATENTSCHRIFT

(11) **EP 2 812 307 B1**
(45) Veröffentlichungstag und Bekanntmachung des Hinweises auf die Patenterteilung: **14.03.2018**
(21) Anmeldenummer: 13701905.5
(22) Anmeldetag: 18.01.2013
(51) Int. Cl.: C07C 67/10, C07C 69/01, C07C 69/24, C07C 69/78, C07C 51/09, C07C 53/08, C07C 53/122, C07C 67/54, C07C 67/055, C07C 67/08, C07C 29/149, C07C 31/08, C07C 31/10, C07C 51/02, C07C 53/12

(54) **VERFAHREN ZUR HERSTELLUNG VON VINYLESTERN**
METHOD FOR PRODUCING VINYL ESTERS
PROCÉDÉ DE PRÉPARATION D'ESTERS DE VINYLE

(30) Priorität: 06.02.2012 DE 102012002282
(43) Veröffentlichungstag der Anmeldung: 17.12.2014
(73) Patentinhaber: OXEA GmbH, 46147 Oberhausen (DE)
(72) Erfinder: JOHNEN, Leif, 46286 Dorsten (DE); STRUTZ, Heinz, 47445 Moers (DE); SCHALAPSKI, Kurt, 46147 Oberhausen (DE); NOWOTNY, Norman, 45276 Essen (DE); HÖFS, Wolfgang, 46147 Oberhausen (DE); GEISEL, Sebastian, 45147 Essen (DE)
(86) Internationale Anmeldenummer: PCT/EP2013/000141
(87) Internationale Veröffentlichungsnummer: WO 2013/117294

(56) Entgegenhaltungen:
- EP-A1- 0 054 158
- EP-A2- 0 376 075
- WO-A1-92/09554
- WO-A1-2011/139360
- US-A- 4 425 277

## Beschreibung

Die vorliegende Erfindung betrifft ein katalytisches Verfahren für die kontinuierliche Herstellung eines Vinylesters einer Carbonsäure durch die Transvinylierungsreaktion eines Vinylesters einer anderen Carbonsäure.

Vinylester von höheren Carbonsäuren besitzen als Comonomere eine gewisse wirtschaftliche Bedeutung. Mit ihnen können die Eigenschaften von Polymeren, wie beispielsweise Polyvinylchlorid, Polyvinylacetat, Polystyrol oder Polyacrylsäureestern modifiziert werden. So kann beispielsweise die Hydrolysebeständigkeit von Dispersionsanstrichen erhöht werden. Auch für die Herstellung von Klebstoffen werden Vinylester von höheren Carbonsäuren eingesetzt. Als technisch bedeutend für diese Einsatzgebiete haben sich Vinylester auf Basis von 2-Ethylhexansäure, Isononansäure, Laurinsäure oder der Versaticsäuren 911, 10 und 1519 von Shell erwiesen. Diese höheren Carbonsäuren sind beispielsweise durch Oxidation von Aldehyden zugänglich, die durch die Oxoreaktion hergestellt werden, oder durch die sogenannte Koch-Synthese aus dem Olefin, Kohlenmonoxid und Wasser. Bei Vinylestern auf Basis der 2-Ethylhexansäure, Laurinsäure oder Isononansäure, falls die Isononansäure überwiegend aus 3,5,5-Trimethylhexansäure besteht, liegen einheitliche Verbindungen vor, während bei Vinylestern der Versaticsäuren 911 Gemische von hoch verzweigten Carbonsäuren mit 9 bis 11 Kohlenstoffatomen und bei Vinylestern der Versaticsäuren 1519 Gemische von hochverzweigten Carbonsäuren mit 15 bis 19 Kohlenstoffatomen im Vinylester gebunden sind. Bei Vinylestern der Versaticsäure 10 sind strukturverschiedene hoch verzweigte Decansäuren wie Neodecansäuren derivatisiert. 3,5,5-Trimethylhexansäure wird durch Hydroformylierung von Di-isobutylen und nachfolgender Oxidation des entsprechenden Aldehyds in technischem Maßstab hergestellt (Ullmanns Encyklopädie der technischen Chemie, 4. Auflage, 1983, Verlag Chemie, Band 9, Seiten 143-145; Band 23, Seiten 606-607).

Es ist bekannt, Vinylester von Carbonsäuren durch Reaktion von Acetylen mit Carbonsäuren herzustellen (G. Hübner, Fette, Seifen, Anstrichmittel 68, 290 (1966), Ullmanns Encyklopädie der technischen Chemie, 4. Auflage, 1983, Verlag Chemie, Band 23, Seiten 606-607). Diese Verfahrensweise wird in EP 1 057 525 A2 aufgegriffen, nach der gasförmiges Acetylen mit der zu vinylierenden Carbonsäure in Gegenwart eines Katalysators in einem Rohrreaktor zur Reaktion gebracht wird. Bei dem bekannten Mehrphasenverfahren bildet die Carbonsäure, die den Katalysator, beispielsweise ein Zinksalz, gelöst enthält, die kontinuierliche Phase, in der gasförmiges Acetylen als dispergierte Phase vorliegt. Der Rohrreaktor wird bei einem Belastungsfaktor von größer 0,8 betrieben. Die Verwendung von Acetylen als Rohstoff erfordert im industriellen Betrieb jedoch einen hohen apparativen und sicherheitstechnischen Aufwand und Acetylen ist zudem im Allgemeinen nur lokal verfügbar.

Es ist ebenfalls bekannt, Vinylester von Carbonsäuren durch die sogenannte Transvinylierungsreaktion mit einem Vinylester einer anderen Carbonsäure herzustellen: 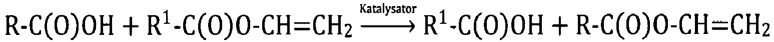 wobei R und R¹ einen aliphatischen oder aromatischen Rest bedeuten können. Um die Gleichgewichtsreaktion in Richtung der Produkte zu steuern, verwendet man häufig einen hohen Überschuss an dem Transvinylierungsreagenz R¹-C(O)O-CH = CH₂. Auch sollte die entstehende Carbonsäure R¹-C(O)OH ausreichend flüchtig sein, um dem Gleichgewicht rasch entzogen zu werden und damit einen erhöhten Umsatz zu generieren. Da das Reaktionsgemisch im Allgemeinen durch Destillation aufgearbeitet wird, richtet sich die Wahl des Transvinylierungsreagenz R¹-C(O)O-CH = CH₂ häufig nach den Siedepunkten der übrigen Reaktionsteilnehmer (Ullmanns Encyklopädie der technischen Chemie, 4. Auflage, 1983, Verlag Chemie, Band 23, Seiten 606-607). Für die Herstellung von Vinylestern höherer Carbonsäuren eignet sich besonders Vinylacetat und in gewissem Maße Vinylpropionat als Transvinylierungsreagenz. Vinylacetat steht als großtechnisch produzierte Chemikalie kostengünstig zur Verfügung. Vinylacetat und die daraus gebildete Essigsäure weisen vergleichsweise geringe Siedepunkte auf und können von dem gewünschten Vinylester der höheren Carbonsäure destillativ abgetrennt werden.

Über die Transvinylierungsreaktion von Carbonsäuren mit Vinylacetat als Vinylierungsreagenz finden sich in der Literatur zahlreiche Hinweise. Adelman, Journal Organic Chemistry, 1949, 14, Seiten 1057-1077 untersucht die Transvinylierung höherer Carbonsäuren, wie Stearinsäure, Laurinsäure oder 3,5,5-Trimethylhexansäure mit Vinylacetat in Gegenwart von Quecksilbersalzen als Katalysator. US 2,245,131 behandelt die Umsetzung von Benzoesäure oder Crotonsäure mit Vinylacetat in Gegenwart von Quecksilberacetat. Das Reaktionsgemisch wird zunächst unter Rückfluss gehalten. Anschließend wird die Reaktionstemperatur erhöht und die gebildete Essigsäure abgetrennt. Das gebildete Vinylbenzoat wird dann in einer weiteren fraktionierten Destillation gereinigt. US 2,299,862 offenbart die Herstellung von Vinyl-2-ethylhexanoat ausgehend von 2-Ethylhexansäure und Vinylacetat in Gegenwart von Quecksilberacetat und Schwefelsäure. Das erhaltene Rohgemisch wird zunächst mit Natriumacetat neutralisiert und anschließend destilliert. Vinyl-2-ethylhexanoat wird mit einer Ausbeute von 73% erhalten. Nach DE 1222493 B verwendet man als Katalysator für die Transvinylierung mit Vinylacetat Quecksilbersalze eines Sulfonsäure-Kationenaustauscherharzes.

Nachteilig bei den Transvinylierungsverfahren mit quecksilberhaltigen Katalysatoren ist ihre Giftigkeit und Flüchtigkeit sowie die unerwünschte Bildung von Ethylidendiestern. Auch die Aktivierung üblicherweise mit Schwefelsäure und eine notwendige Desaktivierung des Katalysators vor der Destillation des Reaktionsgemisches durch Neutralisation bedeuten zusätzliche Verfahrensschritte.

Diese Nachteile lassen sich durch die Anwendung von Palladiumkatalysatoren umgehen, wobei sich die Modifizierung der Palladiumkomplexe mit aromatischen, stickstoffhaltigen Liganden, wie zum Beispiel mit 2,2'-Bipyridyl oder 1,10-Phenanthrolin, als vorteilhaft gezeigt hat. Nach US 5,214,172 wird die Aktivität derartig modifizierter Palladiumkatalysatoren durch den Zusatz starker Säuren erhöht. US 5,741,925 behandelt die Transvinylierung von Naphthensäuren in Gegenwart von Palladiumkomplexen modifiziert mit 2,2'-Bipyridyl oder 1,10-Phenanthrolin. Entsprechend der bekannten Arbeitsweise werden Naphthensäuren, vorzugsweise cyclische C₁₀-C₂₀ Carbonsäuren, zu den entsprechenden Vinylestern mit Vinylacetat als Transvinylierungsreagenz unter Rückfluss umgesetzt. Der Katalysator ist während der Destillation stabil und kann in mehreren Durchläufen erneut eingesetzt werden. Das gemäß US 5,223,621 offenbarte Verfahren betrifft die Transvinylierung von Carbonsäuren, beispielsweise von Laurinsäure oder Benzoesäure, mit einem in situ gebildeten (2,2'-Bipyridyl)palladium(II)diacetat-Komplex unter Rückfluss. Der Katalysator wird vor der Destillation des Rohprodukts mit Oxalsäure oder Salzsäure ausgefällt und abfiltriert.

Auch der Einsatz eines kombinierten Katalysatorsystems aus einem Palladiumsalz und einem Redoxmittel zur Transvinylierung von Carbonsäuren ist bekannt. EP 0 376 075 A2 empfiehlt ein redoxaktives Katalysatorsystem aus Palladiumchlorid, Kupfer(II)bromid und Lithiumacetat. Beispielhaft wird die diskontinuierliche Transvinylierung von Laurinsäure mit Vinylacetat nahe am Siedepunkt des Vinylacetats beschrieben. Der gewünschte Vinylester wird bei einer anschließenden Destillation rein erhalten. Eine weitere Ausgestaltung eines redoxaktiven Katalysatorsystems wird in JP 2002-322125 A offenbart. Dabei wird das Reaktionsgemisch aus Carbonsäure und Vinylacetat sowie Palladiumacetat und Lithiumacetat auf 65°C erhitzt.

Ebenfalls erwähnt der Stand der Technik den Einsatz von rutheniumhaltigen Katalysatoren für die Transvinylierungsreaktion. Nach Murray, Catalysis Today 1992, 13, Seiten 93-102 können höhere Carbonsäuren wie 2-Ethylhexansäure, Benzoesäure, Neodecansäure, Neononansäure oder Adipinsäure mit Vinylacetat in Gegenwart von metallischem Ruthenium oder Rutheniumverbindungen wie Rutheniumchlorid, Rutheniumoxid oder Rutheniumcarbonylen wie Ru₃(CO)₁₂ in die entsprechenden Vinylester umgewandelt werden. Dabei wird die Reaktion diskontinuierlich unter Kohlenmonoxid oder Stickstoff bei einem Druck von etwa 2 bar und einer Temperatur von üblicherweise 130 bis 150°C durchgeführt. Ein entsprechendes Verfahren ist ebenfalls aus EP 0 351 603 A2 und EP 0 506 070 A2 bekannt. Es wird darauf hingewiesen, dass Rutheniumkatalysatoren thermisch stabiler sind als Palladiumkatalysatoren, die bei erhöhten Temperaturen unter Abscheidung von metallischem Palladium desaktivieren. Allerdings werden bei den bekannten rutheniumkatalysierten Verfahren nur mäßige Ausbeuten angegeben.

Die im Stand der Technik überwiegend beschriebenen Verfahren zur Transvinylierungsreaktion werden diskontinuierlich durchgeführt, meistens unter Rückfluss und gelegentlich unter Druck im geschlossenen Reaktionsgefäß.

Ein kontinuierlich betriebenes Transvinylierungsverfahren ist aus EP 0 497 340 A2 bekannt. Mittels einer kontinuierlich betriebenen Reaktivdestillation wird durch kontinuierliches Entfernen der flüchtigsten Reaktionskomponente das Gleichgewicht der Transvinylierungsreaktion R-C(O)OH + R¹-C(O)O-CH = CH₂ → R¹-C(O)OH + R-C(O)O-CH = CH₂ in Richtung der Produkte verschoben. Das Transvinylierungsreagenz R¹-C(O)O-CH = CH₂ wird so gewählt, dass die entsprechende Säure R¹-C(O)OH flüchtig ist und aus dem Gleichgewicht entfernt wird. Das Verfahren nach EP 0 497 340 A2 verwendet als Transvinylierungsreagenz vorzugsweise Vinylacetat und die gebildete Essigsäure wird zusammen mit nicht reagiertem Vinylacetat aus der Reaktionszone entfernt. Anschließend wird das von der Essigsäure in einem separaten Schritt abgetrennte Vinylacetat wieder in die Reaktionszone zurückgeführt. Das bekannte Verfahren arbeitet mit Rutheniumkatalysatoren, beispielsweise mit [Ru(CO)₂OAc]ₙ, und beschreibt die Transvinylierung von Adipinsäure, Neodecansäure und 2-Ethylhexansäure. Um jedoch die unerwünschte Bildung von Säureanhydriden zurückzudrängen, wird nur bis zu einem Teilumsatz an dem gewünschten Vinylester gefahren.

WO 2011/139360 A1 und WO 2011/139361 A1 offenbaren ein kontinuierliches und halbkontinuierliches Transvinylierungsverfahren von Carbonsäuren mit Vinylacetat, wobei Palladium-Komplexe, die aromatische, stickstoffhaltige Liganden enthalten, wie 2,2'-Bipyridyl und 1,10-Phenanthrolin, verwendet werden. Das kontinuierliche Verfahren wird in einer Blasensäule mit aufgesetzter gepackter Säule betrieben, der noch zusätzlich eine Rektifikationssäule und eine Stripp-Säule nachgeschaltet sind. Vinylacetat wird in die Blasensäule eingeleitet während gleichzeitig ein Gemisch aus der Carbonsäure und Vinylacetat, das den Katalysator gelöst enthält, in die aufgesetzte gepackte Kolonne gegeben wird. Carbonsäure und Katalysator fließen in die Blasensäule, während Vinylacetat im Gegenstrom durch die Blasensäule und durch die aufgesetzte gepackte Kolonne geführt wird. Vinylacetat und gebildete Essigsäure werden abgeführt und in einer nachgeschalteten Rektifikationssäule und Stripp-Säule aufgetrennt.

WO92/09554 A1 behandelt ein Verfahren zur Herstellung von Vinylestern in Gegenwart von Rutheniumkatalysatoren, wobei das erhaltene Reaktionsgemisch unter Einsatz eines Azeotropbildners aufgearbeitet wird. Das Verfahren kann kontinuierlich, halb-kontinuierlich, absatzweise oder halb-absatzweise durchgeführt werden.

Aus US 4425277 ist ein kontinuierliches Verfahren zur Herstellung von Vinylestern bekannt. Die Umsetzung erfolgt in Gegenwart eines geträgerten Palladiumkatalysators, der mit einem Co-Katalysator aus einer Alkalimetallverbindung und einer Kupfer (II)-Verbindung versetzt ist.

EP 0 054 158 A1 betrifft ebenfalls ein kontinuierliches Verfahren zur Herstellung von Vinylestern. Die Umsetzung erfolgt in Gegenwart eines Trägerkatalysators auf Basis von Palladium-(II)-Salzen mit Aktivkohle als Trägermaterial mit einem bestimmten analytischen SiO₂-Gehalt.

Nachteilig der im Stand der Technik beschriebenen kontinuierlichen Verfahren ist, dass das Transvinylierungsreagenz Vinylacetat nicht effizient zur Herstellung des Vinylesters genutzt wird, weil es aufgrund der gewählten Reaktionsbedingungen nach sehr kurzer Verweilzeit verdampft und dadurch der Transvinylierungsreaktion nicht mehr zur Verfügung steht. Ferner sind die bekannten kontinuierlich betriebenen Transvinylierungsverfahren apparativ aufwendig und mit hohen Investitionskosten auszulegen, da dem Reaktionsgefäß eine Reihe von Kolonnen nachzuschalten sind, die als zusätzliche Reaktionskolonnen arbeiten. Die bekannten Verfahren erlauben daher nur mäßige Raum-Zeit-Ausbeuten an dem gewünschten Vinylester.

Es besteht daher ein Bedarf an einem kontinuierlich betriebenen Verfahren zur Transvinylierung von Carbonsäuren, dass mit geringem apparativen Aufwand einfach durchzuführen ist und eine hohe Raum-Zeit-Ausbeute, also eine hohe Produktausbringung pro Reaktionsvolumen und Zeit, an dem gewünschten Vinylester ermöglicht. Ebenfalls soll der gewünschte Vinylester mit einer hohen Selektivität gebildet werden.

Die vorliegende Erfindung besteht daher in einem kontinuierlichen, katalytischen Verfahren zur Herstellung eines Vinylesters der Formel R-C(O)O-CH = CH₂ durch Umsetzung einer Carbonsäure der Formel R-C(O)OH mit einem Transvinylierungsreagenz der Formel R¹-C(O)O-CH = CH₂, wobei R und R¹ unabhängig voneinander einen aliphatischen, cycloaliphatischen oder aromatischen Rest bedeuten, dadurch gekennzeichnet, dass die Umsetzung bei einer Temperatur von 90 bis 160°C und bei einem Druck von 0,8 bis 8 MPa ohne Entzug eines Reaktionspartners in Gegenwart eines Übergangsmetallkatalysators enthaltend mindestens ein Übergangsmetall ausgewählt aus der Gruppe von Ruthenium, Osmium, Rhodium, Iridium, Palladium und Platin erfolgt und anschließend das erhaltene Reaktionsgemisch in seine Bestandteile aufgetrennt wird.

Im Gegensatz zu den bekannten kontinuierlich betriebenen Verfahren, bei denen kontinuierlich mindestens ein Reaktionspartner dem Transvinylierungsgleichgewicht entzogen wird und damit das chemische Gleichgewicht ständig gestört wird, wird bei dem erfindungsgemäßen Verfahren die Umsetzung ohne Entzug eines Reaktionspartners im stationären Zustand durchgeführt. Das Reaktionssystem befindet sich im stationären Zustand und das dem Reaktionsgefäß entnommene Gemisch wird erst bei der anschließenden Aufarbeitung in seine Bestandteile aufgetrennt. Die Umsetzung der Carbonsäure R-C(O)OH mit dem Transvinylierungsreagenz R¹-C(O)O-CH = CH₂ erfolgt in Gegenwart eines Übergangsmetallkatalysators und wird bei Temperaturen von 90 bis 160°C, vorzugsweise von 90 bis 150°C und insbesondere von 90 bis 140°C durchgeführt. Obwohl das erfindungsgemäße Verfahren ohne Entzug eines Reaktionspartners betrieben wird, können dennoch überraschenderweise eine hohe Ausbeute und eine hohe Raum-Zeit-Ausbeute an dem gewünschten Vinylester erzielt werden.

Die Transvinylierungsreaktion wird bei einem Druck von 0,8 bis 8 MPa und insbesondere von 0,8 bis 2 MPa durchgeführt. Als ganz besonders geeignet haben sich Reaktionseinstellungen bei einer Temperatur von 90 bis 140°C und bei einem Druck von 0,8 bis 2 MPa erwiesen. Es werden aber auch bei Normaldruck und insbesondere bei einer Reaktionstemperatur von 90 bis 150°C ebenfalls sehr hohe Raum-Zeit-Ausbeuten an dem gewünschten Vinylester erzielt.

Als Reaktionsgefäß eignet sich ein Rohrreaktor, wie ein beliebig angeordnetes Strömungsrohr, zum Beispiel ein senkrecht stehendes oder ein waagerecht angeordnetes Strömungsrohr oder ein mehrfach geschlängeltes Strömungsrohr. Der Rohrreaktor kann als Leerrohr betrieben werden, er kann jedoch ebenfalls Füllkörper oder Einbauten enthalten, beispielsweise Raschigringe, Sättel, Pallringe, Wendel, Strombrecher oder statische Mischer oder Mischerpackungen. Statische Mischelemente stehen kommerziell zur Verfügung und werden beispielsweise als Sulzermischer oder Kenicksmischer angeboten mit speziellen Produktlinien für das Vermischen unterschiedlich viskoser Flüssigkeiten. Der Rohrreaktor kann ebenfalls mit einem Umpump und gegebenenfalls mit einem Wärmetauscher versehen werden.

Bei dem Betrieb des Rohrreaktors können die Einsatzcarbonsäure R-C(O)OH und das Transvinylierungsreagenz R¹-C(O)O-CH = CH₂ getrennt aber gleichzeitig im Gegenstrom oder Gleichstrom in den Rohrreaktor eineingeleitet werden. Es ist aber auch möglich, beide Flüssigkeiten vorab zu vermischen und als homogene Lösung auf den Rohrreaktor zu geben. In einer besonderen Ausführungsform wird die homogene Lösung vor Eintritt in den Rohrreaktor durch ein vorgeschaltetes statisches Mischelement geführt.

Ebenfalls kann die Transvinylierungsreaktion kontinuierlich in einem Rührkessel oder in einer Rührkesselkaskade unter Druck durchgeführt werden. Die Einsatzcarbonsäure R-C(O)OH und das Transvinylierungsreagenz R¹-C(O)O-CH = CH₂ werden entweder getrennt oder als Gemisch kontinuierlich eingespeist und das im stationären Zustand befindliche Reaktionsgemisch wird kontinuierlich abgeführt. Ebenfalls ist die kontinuierliche Reaktionsführung in fachüblichen Reaktorausführungen, wie in einem Schlaufenreaktor unter Nutzung der Wärmekonvektion oder in einem Mehrkammerreaktor möglich. Das Reaktionsgefäß kann ebenfalls als zylindrischer Reaktor mit einer axial angeordneten Düse für den Einlass des flüssigen, katalysatorhaltigen Gemisches aus der Einsatzcarbonsäure R-C(O)OH und dem Transvinylierungsreagenz R¹-C(O)O-CH = CH₂ ausgestaltet werden, der zusätzlich noch zur Erzeugung einer internen Zwangsströmung ein axial angeordnetes Leitrohr enthält.

Es hat sich eine Belastung des Reaktionsgefäßes V/Vh mit einem zuvor hergestellten Gemisch aus dem Transvinylierungsreagenz und der zu vinylierenden Einsatzcarbonsäure von 0,4 bis 7,0 h⁻¹, vorzugsweise von 0,7 bis
6,2 h⁻¹, bezogen auf das Reaktorvolumen und Zeit, als vorteilhaft erwiesen. Werden beide Edukte getrennt aber gleichzeitig in das Reaktionsgefäß eingeleitet, beträgt die Belastung V/Vh des Reaktionsgefäßes mit dem Transvinylierungsreagenz von 0,2 bis 6,0 h⁻¹ und mit der zu vinylierenden Einsatzcarbonsäure von 0,1 bis 6,7 h⁻¹, jeweils bezogen auf das Reaktorvolumen und Zeit.

Durch die im stationären Zustand durchgeführte kontinuierliche Verfahrensführung können vorteilhafterweise sehr hohe Raum-Zeit-Ausbeuten erzielt werden gegenüber der bekannten Reaktivdestillation, bei der kontinuierlich die gebildete Carbonsäure zusammen mit dem Transvinylierungsreagenz aus dem Reaktionssystem entfernt wird. Diese Verfahrensführung erfordert zudem eine hohe Einsatzmenge an dem Transvinylierungsreagenz, um ständig eine ausreichend hohe Konzentration im Reaktionsgemisch zu gewährleisten und um die Reaktivdestillationskolonne ausreichend zu belasten, und eine hohe Kreislaufführung für das Transvinylierungsreagenz ist notwendig. Die bekannte kontinuierliche Reaktionsführung benötigt ein hohes Reaktorvolumen und einen hohen apparativen Aufwand. Daher werden nur mäßige Raum-Zeit-Ausbeuten mit einem vergleichsweise hohen apparativen Aufwand verbunden mit hohen Investitionskosten erzielt.

Zur Vermeidung von Nebenreaktionen, wie der Polymerisation der Vinylester, kann vor Eintritt in das Reaktionsgefäß dem Transvinylierungsreagenz ein geeigneter Inhibitor, wie Hydrochinon, Methoxyhydrochinon, tertiär-Butylcatechol oder Phenothiazin zugesetzt werden. Es ist aber auch möglich, den Inhibitor separat kontinuierlich in das Reaktionsgefäß einzuleiten. Die Konzentration des Inhibitors in der homogenen Reaktionsmischung beträgt im Allgemeinen von 3 bis 150 ppm, bezogen auf die eingesetzte Menge des Transvinylierungsreagenz.

Als Einsatzcarbonsäuren R-C(O)OH, die nach dem erfindungsgemäßen Verfahren in den entsprechenden Vinylester überführt werden können, eignen sich aliphatische, cycloaliphatische oder aromatische Carbonsäuren. Der organische Rest R enthält üblicherweise 2 bis 20 Kohlenstoffatome, vorzugsweise 4 bis 13. Zu den aliphatischen Carbonsäuren zählen beispielsweise Propionsäure, n-Buttersäure, iso-Buttersäure, n-Valeriansäure, 2-Methylbuttersäure, 3-Methylbuttersäure, Pivalinsäure, n-Heptansäure, 2-Methylhexansäure, 2-Ethylhexansäure, n-Octansäure, n-Nonansäure, Isononansäure, Neononansäure, n-Decansäure, 2-Propylheptansäure, Neodecansäure oder Versaticsäure 10, Versaticsäuren 911, Versaticsäuren 1519, Laurinsäure, Tridecansäure, Palmitinsäure oder Stearinsäure. Von den verschiedenen Isononansäuren, die nach dem erfindungsgemäßen Verfahren transvinyliert werden können, ist die 3,5,5-Trimethylhexansäure zu nennen, die durch Hydroformylierung von Di-isobutylen zu dem entsprechenden 3,5,5- Trimethylhexanal und nachfolgender Oxidation zugänglich ist. Wird Di-isobutylen mit Kohlenmonoxid und Wasser in Gegenwart stark saurer Katalysatoren umgesetzt, erhält man überwiegend 2,2,4,4-Tetramethylpentansäure, die auch als Neononansäure bezeichnet wird. Pivalinsäure, Neononansäure, Neodecansäure oder Versaticsäure 10, oder die Versaticsäuren 911, ein Gemisch isomerer C9- bis C11-Carbonsäuren, und Versaticsäuren 1519, ein Gemisch isomerer C15- bis C19-Carbonsäuren, sind hochverzweigte Carbonsäuren, die an dem der Carboxylgruppe benachbarten Kohlenstoffatom drei Alkylreste tragen und eine sogenannte Neo-Struktur besitzen. Trotz der hohen Verzweigung in der Nähe der Carboxylgruppe können diese Neosäuren mit sehr guten Ausbeuten in die entsprechenden Vinylester überführt werden. Versaticsäure ist ein Markenname der Shell.

Daneben können auch aromatische Carbonsäuren, wie Benzoesäure oder Naphthalincarbonsäure, oder ungesättigte aliphatische Carbonsäuren, wie Acrylsäure, Crotonsäure oder Methacrylsäure in das Vinylderivat überführt werden. Flüssige Carbonsäuren können direkt in dem erfindungsgemäßen Verfahren eingesetzt werden. Feste Carbonsäuren werden in einem Lösungsmittel, beispielsweise in Toluol, THF, Dioxan oder cyclischen Ethern, oder direkt in dem Transvinylierungsreagenz gelöst und als Lösung in die Transvinylierungsreaktion eingesetzt.
Als Transvinylierungsreagenz R¹C(O)O-CH = CH₂ eignen sich sämtliche Vinylester, die im industriellen Maßstab kostengünstig in großen Mengen zur Verfügung stehen. Im Allgemeinen bedeutet R¹ einen aliphatischen, cycloaliphatischen oder aromatischen Rest. Der organische Rest R¹ enthält üblicherweise 1 bis 20 Kohlenstoffatome. Bevorzugt verwendet man Vinylacetat mit R¹ gleich Methyl oder Vinylpropionat mit R¹ gleich Ethyl aber auch Vinylester höherer Carbonsäuren wie Vinyllaurat mit R¹ gleich Undecyl.

Die Wahl des Transvinylierungsreagenz hängt von den physikalischen Eigenschaften der zu derivatisierenden Carbonsäure R-C(O)OH und den gebildeten Reaktionspartnern R¹-C(O)OH und R-C(O)O-CH = CH₂ ab und richtet sich danach, durch welche Verfahren das Reaktionsgemisch in die verschiedenen Komponenten zweckmäßigerweise aufgetrennt werden kann. Üblicherweise werden das zurückgewonnene Transvinylierungsreagenz R¹-C(O)O-CH = CH₂ und die zurückgewonnene Einsatzcarbonsäure R-C(O)OH wieder in die Transvinylierungsreaktion zurückgeführt. Die durch die Transvinylierungsreakion gebildete Carbonsäure R¹-C(O)OH wird im Zuge der Aufarbeitung des Reaktionsgemisches abgetrennt und der gewünschte Vinylester R-C(O)O-CH = CH₂ weiter aufgereinigt. Üblicherweise wird das erhaltene Reaktionsgemisch durch Destillation aufgearbeitet, eine Aufarbeitung mittels Kristallisation oder Ausfällung ist nicht ausgeschlossen, aber auf bestimmte Sonderfälle beschränkt.

Es hat sich als zweckmäßig erwiesen, bei der Aufarbeitung des Reaktionsgemisches und bei der weiteren Aufreinigung des gewünschten Vinylesters ebenfalls einen Inhibitor, wie Hydrochinon, Methoxyhydrochinon, tertiär-Butylcatechol oder Phenothiazin zuzusetzen. Der bei der Aufarbeitung des Reaktionsgemisches anfallende Transvinylierungskatalysator wird anschließend wieder in die Transvinylierungsreaktion zurückgeführt, gegebenenfalls nach Zugabe von Frischkatalyator oder frischen Liganden.

Bezogen auf Carbonsäureeinsatz R-C(O)OH kann das Transvinylierungsreagenz R¹-C(O)O-CH = CH₂ in einem molaren Verhältnis von 0,1 zu 1 bis 10 zu 1, vorzugsweise von 0,2 zu 1 bis 5 zu 1, eingesetzt werden. Seine Menge richtet sich ebenfalls nach den physikalischen Eigenschaften der Einsatzstoffe und den gebildeten Reaktionspartnern und danach, auf welche Weise das Reaktionsgemisch möglichst vorteilhaft aufgearbeitet werden kann.

Vinylacetat hat sich aufgrund der kostengünstigen Verfügbarkeit, seines Siedepunktes und des Siedepunktes der in der Transvinylierungsreaktion gebildeten Essigsäure als vorteilhaftes Transvinylierungsreagenz erwiesen, insbesondere bei der Transvinylierung von Carbonsäuren mit fünf und mehr Kohlenstoffatomen, wie zum Beispiel von n-Valeriansäure, 2-Methylbuttersäure, 3-Methylbuttersäure, Pivalinsäure, n-Heptansäure, 2-Methylhexansäure, n-Octansäure, 2-Ethylhexansäure, n-Nonansäure, Isononansäure, Neononansäure, 3,5,5-Trimethylhexansäure, n-Decansäure, 2-Propylheptansäure, Neodecansäure oder Versaticsäure 10, Versaticsäuren 911, Versaticsäuren 1519, Laurinsäure, Tridecansäure, Palmitinsäure, Stearinsäure oder Benzoesäure oder Naphthalincarbonsäure. Aber auch die Transvinylierung von niederen Carbonsäuren, wie Propionsäure, n-Buttersäure, iso-Buttersäure, Acrylsäure, Crotonsäure oder Methacrylsäure ist nach dem erfindungsgemäßen Verfahren mit Vinylacetat möglich. Bezogen auf den molaren Carbonsäureeinsatz wird Vinylacetat üblicherweise im molaren Überschuss von bis zu 10 zu 1, vorzugsweise bis zu 5 zu 1 eingesetzt. Das Reaktionsgemisch wird üblicherweise destillativ aufgearbeitet. Überschüssiges Vinylacetat, gebildete Essigsäure und der gewünschte Vinylester werden als flüchtige Komponenten abgezogen und weiter aufgetrennt. Im Rückstand verbleibt die Einsatzcarbonsäure zusammen mit dem Transvinylierungskatalysator. Der katalysatorhaltige Rückstand wird nach optionalem Ausschleusen eines hochsiederhaltigen Teilstromes wieder in die Transvinylierungsreaktion zurückgeführt, gegebenenfalls nach Zugabe von Frischkatalysator oder frischen Liganden.

Es ist ebenfalls möglich, Vinylacetat im molaren Unterschuss bis zu 0,1 zu 1, vorzugsweise bis zu 0,2 zu 1, bezogen auf den molaren Carbonsäureeinsatz zu verwenden. Dadurch kann der Aufwand zur Abtrennung des Vinylacetats verringert werden.

Als Transvinylierungskatalysator werden Komplexverbindungen der Übergangsmetalle aus der Platingruppe Ruthenium, Osmium, Rhodium, Iridium, Palladium und Platin eingesetzt, die mit ein- oder mehrzähnigen Organostickstoff- oder Organophosphorliganden modifiziert sind. Die Gesamtkonzentration des Übergangsmetalls oder der Übergangsmetalle, wenn eine Mischung davon verwendet wird, beträgt im Allgemeinen von 0,005 bis 1,5 mol-%, vorzugsweise von 0,01 bis 1,0 und insbesondere von 0,02 bis 0,6 mol-%, jeweils bezogen auf die im Unterschuss eingesetzte Ausgangsverbindung, also bezogen auf die eingesetzte Carbonsäure R-C(O)OH oder bezogen auf das eingesetzte Transvinylierungsreagenz R¹C(O)O-CH=CH₂. Obgleich es möglich ist, als Katalysator die stöchiometrisch zusammengesetzte Übergangsmetall-Ligand-Komplexverbindung einzusetzen, arbeitet man üblicherweise in Gegenwart von überschüssigen Liganden, d. h. Ligand, der mit dem Übergangsmetall keine komplexe Bindung eingegangen ist. Je mol Übergangsmetall wendet man 1 bis 40, vorzugsweise 3 bis 30 mol Ligand an. Besonders bewährt haben sich molare Verhältnisse von Übergangsmetall zu Ligand im Bereich von 1:3 bis 1:10. Als einzähnige Organostickstoffliganden eignen sich beispielsweise Pyridin, Chinolin, die stellungsisomeren Pikoline, die stellungsisomeren Lutidine, Collidin, Anilin, die stellungsisomeren Toluidine, die stellungsisomeren Xylidine, N-Methylanilin oder aliphatische und aromatische Amide wie N,N-Dimethylformamid, Acetanilid oder N-Methyl-2-pyrrolidon; als einzähnige Organophosphorliganden eignen sich beispielsweise Trialkylphosphine wie Tributylphosphin oder Trioctylphosphin, Triarylphosphine wie Triphenylphosphin oder Tritolylphosphin, Tricycloalkylphosphine wie Tricyclohexylphosphin, Alkylarylphosphine wie Monobutyldiphenylphosphin oder Dipropylphenylphosphin, Cycloalkylarylphosphine, Trialkylphosphite oder Triarylphosphite wie Triphenylphosphit oder Trinaphthylphosphit. Als mehrzähnige Organostickstoff- oder Organophosphorliganden eignen sich beispielsweise zweizähnige Liganden wie 2,2'-Bipyridyl, 1,10-Phenanthrolin, N,N,N',N'-Tetramethylethylendiamin, P,P,P',P'-Tetraphenyl-1,2-diphosphinoethan, 4,7-Diphenyl-1,10-phenanthrolin, 5-Chloro-1,10-phenanthrolin, 3,4,7,8-Tetramethyl-1,10-phenanthrolin, 2,9,4,7-Tetramethyl-1,10-phenanthrolin, 2,9-Dimethyl-4,7-diphenyl-1,10-phenanthrolin, 2,9-Dimethyl-1,10-phenanthrolin, 4,4'-Diphenyl-2,2'-bipyridyl, 4-Methyl-1,10-phenanthrolin, 2,2'-Bichinolin oder 5-Methyl-1,10-phenanthrolin.

Der Übergangsmetall-Ligand-Komplex braucht nicht einheitlich zusammengesetzt sein, sondern kann zum Beispiel aus einem Gemisch von Übergangsmetall-Ligand-Komplexverbindungen bestehen, die sich in der Art der Liganden und/oder des Übergangsmetalls unterscheiden. Ebenso kann der in der organischen Lösung enthaltene freie Ligand aus einem Gemisch unterschiedlicher ein- oder mehrzähniger Organostickstoff- oder Organophosphorliganden zusammengesetzt sein. Man bildet den Transvinylierungskatalysator üblicherweise aus dem Übergangsmetall, einer Übergangsmetallverbindung oder entsprechender Mischungen und dem ein- oder mehrzähnigen Organostickstoffligand, Organophosphorligand oder entsprechender Mischungen unter den Bedingungen der Transvinylierungsreaktion im Reaktionsgemisch.

Es ist ebenfalls möglich, den Transvinylierungskatalysator gesondert in einer Präformierungsreaktion herzustellen. Als geeignetes Lösungsmittel, in denen die Präformierung durchgeführt wird, kann das Transvinylierungsreagenz, die zu vinylierende Einsatzcarbonsäure oder ein Gemisch davon verwendet werden. Die Bedingungen des Präformierungsschrittes entsprechen im Allgemeinen den Bedingungen der Transvinylierungsreaktion. Die Präformierungsbedingungen können beim Anfahren des Transvinylierungsprozesses eingestellt werden, so dass der Einlass des Transvinylierungsreagenz und der zu vinylierenden Einsatzcarbonsäure in das Reaktionsgefäß erst dann erfolgt, wenn sich der aktive Transvinylierungskatalysator in der vorgelegten organischen Lösung gebildet hat. Wird der Übergangsmetall-Ligand-Komplexkatalysator während des laufenden Prozesses zugesetzt, so ist in einem separaten Präformierungsschritt zunächst eine Lösung des aktiven Übergangsmetall-Ligand-Komplexkatalysator herzustellen, die anschließend zu dem Prozess als Frischlösung ergänzt wird. Dabei verwendet man im Präformierungsschritt als Lösungsmittel das Transvinylierungsreagenz, die zu vinylierende Einsatzcarbonsäure oder eine Mischung davon. Auch während des Präformierungsschrittes kann der Ligand im Überschuss eingesetzt werden, so dass sich während der Transvinylierungsreaktion die zuvor erwähnten Molverhältnisse zwischen Übergangsmetall und Ligand einstellen.

Als Transvinylierungskatalysator können ebenfalls unmodifizierte Übergangsmetallkomplexe enthaltend mindestens ein Übergangsmetall ausgewählt aus der Gruppe von Ruthenium Osmium, Rhodium, Iridium, Palladium und Platin verwendet werden, die keine ein- oder mehrzähnigen Organostickstoff- oder Organophosphorliganden tragen. Es kann angenommen werden, dass sich unter den Bedingungen der Transvinylierungsreaktion der aktive Übergangsmetallkomplex aus den eingesetzten Katalysatorvorstufen, wie den Übergangsmetallen, Übergangsmetallcarbonylen, -carboxylaten, -halogeniden oder -acetylacetonaten bildet. Gegebenenfalls wird beim Einsatz unmodifizierter Übergangsmetallkomplexe Kohlenmonoxid zugesetzt. Besonders Rutheniumkatalysatoren werden in unmodifizierter Form verwendet.

Bei dem Einsatz unmodifizierter Übergangsmetallkomplexe kann es sich als vorteilhaft erweisen, noch ein redoxaktives Übergangsmetall der Gruppe Ib des Periodensystems der Elemente sowie eine Alkalimetallverbindung zuzusetzen. Als redoxaktives Übergangsmetall eignet sich beispielsweise Kupfer in Form von Halogeniden des zweiwertigen Kupfers. Als Alkalimetallverbindungen verwendet man vorzugsweise Lithiumverbindungen, beispielsweise Lithiumcarboxylate, wie Lithiumacetat oder Lithiumpropionat, Lithiumcarbonat, Lithiumhydrogencarbonat, Lithiumchlorid oder Lithiumhydroxid. Ein geeigneter Transvinylierungskatalysator kann beispielsweise aus den Vorstufen Palladiumchlorid, Kupfer(II)bromid und Lithiumacetat gebildet werden. Die Bildung des aktiven Transvinylierungskatalysators aus den geeigneten Vorstufen erfolgt im Reaktionsgefäß unter den Reaktionsbedingungen. Eine Präformierung des Katalysators, entweder zu Beginn im Reaktionsgefäß oder in einem separaten Gefäß, ist ebenfalls möglich.

Das Übergangsmetall aus der Platingruppe gelangt entweder als Metall oder als Verbindung zum Einsatz. In metallischer Form verwendet man es entweder als feinverteilte Partikel oder in dünner Schicht auf einem Träger, wie Aktivkohle, Calciumcarbonat, Aluminiumsilikat, Tonerde, niedergeschlagen. Als Übergangsmetallverbindungen eignen sich Salze der zu vinylierenden Einsatzcarbonsäure oder Salze der gebildeten entsprechenden Carbonsäure, wie zum Beispiel Acetate, Propionate, Butyrate, 2-Ethylhexanoate oder Isononanoate. Weiterhin können Salze anorganischer Wasserstoff- oder Sauerstoffsäuren, wie Nitrate oder Sulfate, die verschiedenen Oxide oder auch Carbonylverbindungen oder Komplexverbindungen wie Cyclooctadienylverbindungen oder Acetylacetonate eingesetzt werden. Übergangsmetallhalogenverbindungen sind möglich, kommen wegen des korrosiven Verhaltens der Halogenidionen aber weniger in Betracht.

Vorzugsweise verwendet man Palladium- oder Rutheniumverbindungen, insbesondere deren Acetate, Propionate, Butyrate, 2-Ethylhexanoate, Isononanoate, Acetylacetonate, Triflate, Trifluoroacetate oder Carbonyle wie Ru₃(CO)₁₂, H₄Ru₄(CO)₁₂ oder [Ru(CO)₂OAc]ₙ.

Der flüssige Austrag aus dem Reaktionsgefäß wird anschließend über Druckentspannungsstufen auf Normaldruck entspannt und in weiteren Trenneinrichtungen aufgearbeitet. Es kann sich auch als zweckmäßig erweisen, den flüssigen Reaktionsaustrag zunächst auf eine solche Temperatur abzukühlen, dass die Bildung gasförmiger Produkte in der Entspannungsstufe gemindert wird. Dabei gegebenenfalls gebildete gasförmige Anteile werden abgetrennt und die erhaltene flüssige Phase weiter aufgearbeitet. Das flüssige Reaktionsgemisch enthaltend das Transvinylierungsreagenz, die gebildete Carbonsäure, den gewünschten Vinylester, die Einsatzcarbonsäure und den Transvinylierungskatalysator wird getrennt und in die einzelnen Produktströme aufgespalten, wobei zurückgewonnenes Transvinylierungsreagenz üblicherweise wieder in das Reaktionsgefäß zurückgeführt wird und die gebildete Carbonsäure aus dem Prozess entfernt wird und der gewünschte Vinylester weiter aufgereinigt wird. Der Transvinylierungskatalysator sammelt sich in der am wenigsten flüchtigen Reaktionskomponente an und wird, gegebenenfalls nach Ausschleusen eines hochsiederhaltigen Teilstromes, in das Reaktionsgefäß zurückgeführt. In Abhängigkeit von den physikalischen Eigenschaften der Reaktionspartner kann es sich bei der am wenigsten flüchtigen Reaktionskomponente beispielsweise um die nicht umgesetzte Einsatzcarbonsäure, die gebildete Carbonsäure oder um das Transvinylierungsreagenz handeln. Es ist ebenfalls möglich, den flüssigen Austrag aus dem Reaktionsgefäß direkt auf eine Trenneinrichtung zu geben, die unter Druck betrieben wird, und die erhaltenen Stoffströme getrennt weiterzuverarbeiten.

Im Falle von Vinylacetat als Transvinylierungsreagenz hat es sich als zweckmäßig erwiesen, das Reaktionsgemisch auf Normaldruck zu entspannen und destillativ aufzuarbeiten. Vinylacetat, die gebildete Essigsäure und der gewünschte Vinylester werden als flüchtige Komponenten von der nicht umgesetzten Einsatzcarbonsäure abgetrennt, die den Transvinylierungskatalysator enthält. Die abgezogenen flüchtigen Komponenten werden anschließend destillativ in Vinylacetat, Essigsäure und gewünschten Vinylester aufgetrennt. Vinylacetat wird in das Reaktionsgefäß zurückgeführt, Essigsäure wird ausgeschleust und der gewünschte Vinylester weiter aufgereinigt. Die schwerersiedende katalysatorhaltige Einsatzcarbonsäure wird als Katalysatorkreislauf wieder zurückgeführt. Dem Katalysatorkreislauf wird gegebenenfalls ein hochsiederhaltiger Teilstrom entnommen sowie Frischkatalysator, gegebenenfalls in präformierter Form, öder nur frischer Ligand zugesetzt.

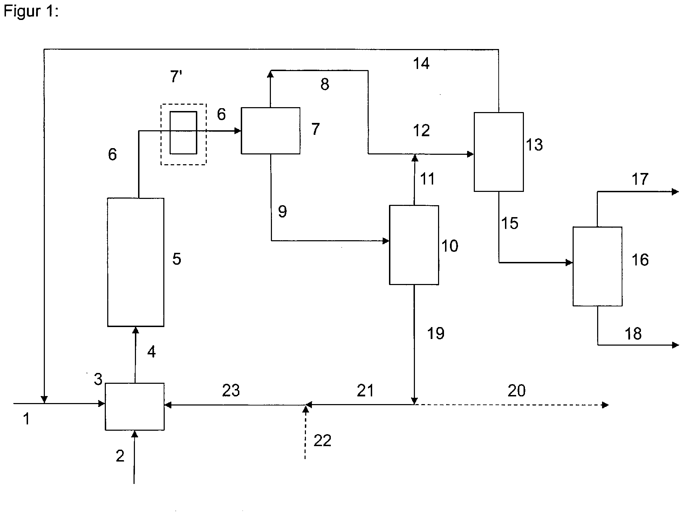

Das bevorzugte erfindungsgemäße Verfahren wird im Folgenden anhand des Prinzipschemas gemäß Figur 1 für den Fall näher erläutert, bei dem die Einsatzcarbonsäure am wenigsten flüchtig ist und bei der Aufarbeitung des Reaktionsgemisches als schwerer flüchtiger Rückstand anfällt. Das erfindungsgemäße Verfahren ist aber nicht auf die in der Zeichnung dargestellte Ausführungsform beschränkt und kann auch mit Erfolg auf solche Ausführungsformen angewandt werden, bei denen irgendein Reaktionspartner als schwerer flüchtige Komponente anfällt.

Das Transvinylierungsreagenz R¹-C(O)O-CH = CH₂ wird über die Leitung (1) und die zu vinylierende Einsatzcarbonsäure R-C(O)OH über die Leitung (2) in ein Mischgefäß (3) geführt, aus dem über Leitung (4) die Mischung in das Reaktionsgefäß (5) eingeleitet wird. Der flüssige Reaktionsaustrag wird über Leitung (6) auf ein Entspannungsgefäß (7) gegeben, in dem auf Normaldruck entspannt wird. Gegebenenfalls wird der flüssige Reaktionsaustrag zunächst in eine Kühleinrichtung (7') (gestrichelt dargestellt) geleitet und gekühlt über die Leitung (6) auf das Entspannungsgefäß (7) gegeben, um alle Komponenten bei Entspannung flüssig zu halten. Die dabei gegebenenfalls gebildete Gasphase wird über Leitung (8) abgeführt und die gebildete Flüssigphase wird über Leitung (9) auf das Trenngefäß (10) gegeben. In dem Trenngefäß (10) erfolgt eine Aufspaltung in eine mit dem Transvinylierungsreagenz R¹-C(O)O-CH = CH₂, der gebildeten Carbonsäure R¹-C(O)OH und dem gewünschten Vinylester R-C(O)O-CH = CH₂ angereicherte flüchtige Fraktion, die über Leitung (11) mit den gegebenenfalls über Leitung (8) herangeführten flüchtigen Anteilen aus der Entspannungsstufe vereinigt wird und über Leitung (12) auf das Trenngefäß (13) geführt wird. Das im Trenngefäß (13) abgetrennte Transvinylierungsreagenz wird über Leitung (14) zurückgeführt und mit dem über Leitung (1) herangeführten Transvinylierungsreagenz R¹-C(O)O-CH = CH₂ vereinigt. Die im Trenngefäß (13) anfallende und während der Transvinylierungsreaktion gebildete Carbonsäure R¹-C(O)OH sowie der gewünschte Vinylester R-C(O)O-CH = CH₂ werden über Leitung (15) abgeführt und auf das Trenngefäß (16) gegeben, aus dem die gebildete Carbonsäure R¹-C(O)OH über Leitung (17) und der gewünschte Vinylester R-C(O)O-CH = CH₂ über Leitung (18) abfließen. Der über Leitung (18) erhaltene Vinylester kann einer weiteren Feinreinigung unterzogen werden (nicht in Figur 1 gezeigt).

Die in dem Trenngefäß (10) erhaltene, schwerer flüchtige Fraktion, die in einer bevorzugten Ausführungsform die nicht umgesetzte Einsatzcarbonsäure R-C(O)OH und den Transvinylierungskatalysator enthält, wird über Leitung (19) abgeführt und gegebenenfalls nach Ausschleusen eines hochsiederhaltigen Seitenstroms über Leitung (20) (gestrichelt dargestellt) als Katalysatorkreislauf über Leitung (21) zurückgeführt. In den Katalysatorkreislauf wird über Leitung (22) gegebenenfalls Frischkatalysator (gestrichelt dargestellt), gegebenenfalls in präformierter Form, oder frischer Ligand ergänzt und die Mischung aus Alt- und Frischkatalysator über Leitung (23) in das Mischgefäß (3) eingespeist. Sowohl im Reaktionsteil als auch im Aufarbeitungsteil kann an geeigneten Stellen Inhibitor zugesetzt werden (nicht in Figur 1 gezeigt). Als Trenngefäße (10), (13) und (16) eignen sich für Trennoperationen übliche Apparate, wie Dünnschichtverdampfer, Kurzwegverdampfer oder Destillationskolonnen. Die einzustellenden Temperatur- und Druckbedingungen richten sich nach den im aufzuarbeitenden Reaktionsgemisch vorliegenden Komponenten und können durch Routineversuche ermittelt werden. Die Auslegung der Trenngefäße, wie die Notwendigkeit und Anzahl der Trennstufen, kann ebenfalls durch Routineversuche oder Simulation ermittelt werden.

Die Erfindung wird in den nachfolgenden Beispielen näher erläutert, jedoch nicht auf die beschriebenen Ausführungsformen beschränkt.

### Beispiele

Für die Durchführung der nachfolgenden Beispiele wurde der Versuchsaufbau gemäß Figur 1 verwendet. In dem Vorlagegefäß (3) wurde über Leitung (1) herangeführtes Vinylacetat (Beispiele 1-7, 15 - 22) oder Vinylpropionat (Beispiele 8-14), über Leitung (2) die zu vinylierende Einsatzcarbonsäure R-C(O)OH und über Leitung (23) Katalysatorlösung vermischt und über Leitung (4) auf das als Strömungsrohr ausgestaltete Reaktionsgefäß (5) gepumpt. Das über Leitung (6) entnommene flüssige Reaktionsgemisch wurde in dem Entspannungsgefäß (7) auf Normaldruck entspannt. Dabei gebildete gasförmige Anteile, die Vinylacetat oder Vinylpropionat und gebildete Essigsäure oder Propionsäure enthielten, wurden über Leitung (8) abgezogen. Der über Leitung (9) abgeführte flüssige Austrag wurde gaschromatographisch analysiert.

Die zur Transvinylierung verwendeten Einsatzcarbonsäuren R-C(O)OH, die im Reaktionsgefäß (5) eingestellten Reaktionsbedingungen sowie die aus der gaschromatographischen Analyse ermittelten Raum-Zeit-Ausbeuten an den gewünschten Vinylestern R-C(O)O-CH = CH₂ sind in den nachfolgenden Tabellen 1-7 zusammengefasst. Die Katalysatorlösung wurde durch Abmischen der Katalysatorvorstufe Palladiumacetat Pd(OAc)₂ mit dem zweizähnigen, stickstoffhaltigen Liganden 1,10-Phenanthrolin (Beispiele 1-16, 21-24) oder 2,2'-Bipyridyl (Beispiele 17, 18) in einer Mischung aus Vinylacetat oder Vinylpropionat und der jeweiligen Einsatzcarbonsäure zubereitet. Bei den Beispielen 19 und 20 verwendete man den Komplex [Ru(CO)₂OAC]ₙ als Katalysatorvorstufe. Bei den Beispielen 21-23 wurde, bezogen auf den molaren Carbonsäureeinsatz, Vinylacetat im molaren Unterschuss eingesetzt. Die Bildung des aktiven Katalysators erfolgte im Reaktionsgefäß unter Reaktionsbedingungen. Die Angabe des molaren Verhältnisses für die Katalysatorvorstufe bezieht sich auf mol Palladium oder mol Ruthenium. Die eingesetzte iso-Nonansäure basierte auf der Hydroformylierung von Di-isobutylen mit nachfolgender Oxidation des entsprechenden Aldehyds und enthielt überwiegend 3,5,5-Trimethylhexansäure.

**Tabelle 1: Bedingungen und Ergebnisse der kontinuierlichen Herstellung von Vinylestern im Strömungsrohr mit Vinylacetat als Transvinylierungsreagenz bei erhöhtem Druck (Pd(OAc)₂/1,10-Phenanthrolin)**

| Beispiel Nr. | 1 | 2 | 3 | 4 | 5 | 6 | 7 |
|---|---|---|---|---|---|---|---|
| Carbonsäure R-C(O)OH | Iso-Nonansäure (3,5,5-Trimethylhexansäure) | Benzoesäure | Pivalinsäure | neo-Decansäure | 2-Ethylhexansäure | Heptansäure | Laurinsäure |
| Verweilzeit [min] | 75 | 75 | 75 | 75 | 75 | 75 | 75 |
| Reaktorvolumen [ml] | 100 | 100 | 100 | 100 | 100 | 100 | 100 |
| Temperatur [°C] | 140 | 140 | 140 | 140 | 140 | 140 | 140 |
| Druck [MPa] | 2 | 2 | 2 | 2 | 2 | 2 | 2 |
| Molares Verhältnis Carbonsäure:Vinylacetat: Katalysatorvorstufe | 1,0:5,0: 0,0010 | 1,0:8,0: 0,0010 | 1,0:5,0: 0,0010 | 1,0:5,0: 0,0010 | 1,0:5,0: 0,0010 | 1,0:5,0: 0,0010 | 1,0:8,0: 0,0010 |
| Molares Verhältnis Katalysatorvorstufe:Liqand | 1:8 | 1:8 | 1:8 | 1:8 | 1:8 | 1:8 | 1:8 |
| Katalysatorvorstufe | Pd(OAc)₂ | Pd(OAc)₂ | Pd(OAc)₂ | Pd(OAc)₂ | Pd(OAc)₂ | Pd(OAc)₂ | Pd(OAc)₂ |
| Ligand | 1,10-Phenanthrolin | 1,10-Phenanthrolin | 1,10-Phenanthrolin | 1,10-Phenanthrolin | 1,10-Phenanthrolin | 1,10-Phenanthrolin | 1,10-Phenanthrolin |
| Carbonsäure [g/h] | 19,8 | 11,7 | 14,2 | 23,4 | 18,5 | 17,2 | 16,6 |
| Vinylacetat [g/h] | 53,9 | 65,8 | 59,9 | 58,4 | 55,4 | 57,0 | 56,9 |
| Katalysatorvorstufe [mg/h] | 28,1 | 21,5 | 31,2 | 30,5 | 28,9 | 29,7 | 18,5 |
| Ligand [mg/h] | 180,6 | 137,8 | 200,6 | 195,6 | 185,4 | 190,8 | 119,1 |
| Umsatz [%] | 83 | 86 | 81 | 72 | 82 | 80 | 74 |
| Ausbeute [%] | 80 | 81 | 74 | 65 | 77 | 76 | 68 |
| Selektivität [%] | 97,0 | 94,4 | 91,3 | 89,9 | 93,5 | 95,0 | 91,9 |
| Raum-Zeit-Ausbeute [g/L·h] | 185 | 115 | 132 | 175 | 168 | 157 | 128 |

**Tabelle 2: Bedingungen und Ergebnisse der kontinuierlichen Herstellung von Vinylestern im Strömungsrohr mit Vinylpropionat als Transvinylierungsreagenz bei erhöhtem Druck (Pd(OAc)₂/1,10-Phenanthrolin)**

| Beispiel Nr. | 8 | 9 | 10 | 11 | 12 | 13 | 14 |
|---|---|---|---|---|---|---|---|
| Carbonsäure R-C(O)OH | Iso-Nonansäure (3,5,5-Trimethylhexansäure) | Benzoesäure | Pivalinsäure | neo-Decansäure | 2-Ethylhexansäure | Heptansäure | Laurinsäure |
| Verweilzeit [min] | 75 | 75 | 75 | 75 | 75 | 75 | 75 |
| Reaktorvolumen [ml] | 100 | 100 | 100 | 100 | 100 | 100 | 100 |
| Temperatur [°C] | 140 | 140 | 140 | 140 | 140 | 140 | 140 |
| Druck [MPa] | 2 | 2 | 2 | 2 | 2 | 2 | 2 |
| Molares Verhältnis Carbonsäure:Vinylpropionat :Katalysatorvorstufe | 1,0:5,0: 0,0010 | 1,0:8,0: 0,0010 | 1,0:5,0: 0,0010 | 1,0:5,0: 0,0010 | 1,0:5,0: 0,0010 | 1,0:5,0: 0,0010 | 1,0:8,0: 0,0010 |
| Molares Verhältnis Katalysatorvorstufe:Ligand | 1:8 | 1:8 | 1:8 | 1:8 | 1:8 | 1:8 | 1:8 |
| Katalysatorvorstufe | Pd(OAc)₂ | Pd(OAc)₂ | Pd(OAc)₂ | Pd(OAc)₂ | Pd(OAc)₂ | Pd(OAc)₂ | Pd(OAc)₂ |
| Ligand | 1,10-Phenanthrolin | 1,10-Phenanthrolin | 1,10-Phenanthrolin | 1,10-Phenanthrolin | 1,10-Phenanthrolin | 1,10-Phenanthrolin | 1,10-Phenanthrolin |
| Carbonsäure [g/h] | 17,6 | 10,1 | 12,4 | 18,8 | 16,4 | 15,2 | 14,6 |
| Vinylpropionat [g/h] | 55,6 | 66,3 | 61,0 | 54,6 | 57,0 | 58,4 | 58,3 |
| Katalysatorvorstufe [mg/h] | 24,9 | 18,6 | 27,4 | 24,5 | 25,6 | 26,2 | 16,4 |
| Ligand [mg/h] | 160,2 | 119,3 | 175,7 | 157,3 | 164,1 | 168,2 | 105,0 |
| Umsatz [%] | 80 | 82 | 77 | 69 | 79 | 76 | 69 |
| Ausbeute [%] | 77 | 77 | 71 | 61 | 75 | 72 | 63 |
| Selektivität [%] | 96,1 | 93,9 | 92,2 | 88,4 | 94,9 | 94,7 | 91,3 |
| Raum-Zeit-Ausbeute [g/L·h] | 158 | 94 | 110 | 132 | 145 | 131 | 104 |

**Tabelle 3: Bedingungen und Ergebnisse der kontinuierlichen Herstellung von Vinylestern im Strömungsrohr mit Vinylacetat als Transvinylierungsreagenz bei Normaldruck (Vergleich) (Pd(OAc)₂/1,10-Phenanthrolin)**

| Beispiel Nr. | 15 (Vergleich) | 16 (Vergleich) |
|---|---|---|
| Carbonsäure R-C(O)OH | Iso-Nonansäure (3,5,5-Trimethylhexansäure) | 2-Ethylhexansäure |
| Verweilzeit [min] | 75 | 75 |
| Reaktorvolumen [ml] | 100 | 100 |
| Temperatur [°C] | 140 | 140 |
| Druck [MPa] | Normaldruck | Normaldruck |
| Molares Verhältnis Carbonsäure:Vinylacetat: Katalysatorvorstufe | 1,0:5,0:0,0010 | 1,0:5,0:0,0010 |
| Molares Verhältnis Katalysatorvorstufe:Ligand | 1:8 | 1:8 |
| Katalysatorvorstufe | Pd(OAc)₂ | Pd(OAc)₂ |
| Ligand | 1,10-Phenanthrolin | 1,10-Phenanthrolin |
| Carbonsäure [g/h] | 19,8 | 18,5 |
| Vinylacetat [g/h] | 53,9 | 55,4 |
| Katalysatorvorstufe [mg/h] | 28,1 | 28,9 |
| Ligand [mg/h] | 180,6 | 185,4 |
| Umsatz [%] | 75 | 79 |
| Ausbeute [%] | 72 | 65 |
| Selektivität [%] | 96,5 | 82,7 |
| Raum-Zeit-Ausbeute [g/L·h] | 167 | 143 |

**Tabelle 4: Bedingungen und Ergebnisse der kontinuierlichen Herstellung von Vinylestern im Strömungsrohr mit Vinylacetat als Transvinylierungsreagenz bei erhöhtem Druck (Pd(OAc)₂/2,2'-Bipyridyl)**

| Beispiel Nr. | 17 | 18 |
|---|---|---|
| Carbonsäure R-C(O)OH | Iso-Nonansäure (3,5,5-Trimethylhexansäure) | 2-Ethylhexansäure |
| Verweilzeit [min] | 75 | 75 |
| Reaktorvolumen [ml] | 100 | 100 |
| Temperatur [°C] | 140 | 140 |
| Druck [MPa] | 2 | 2 |
| Molares Verhältnis Carbonsäure:Vinylacetat: Katalysatorvorstufe | 1,0:5,0:0,0010 | 1,0:5,0:0,0010 |
| Molares Verhältnis Katalysatorvorstufe:Ligand | 1:8 | 1:8 |
| Katalysatorvorstufe | Pd(OAc)₂ | Pd(OAc)₂ |
| Ligand | 2,2'-Bipyridyl) | 2,2'-Bipyridyl |
| Carbonsäure [g/h] | 19,8 | 18,5 |
| Vinylacetat [g/h] | 53,9 | 55,4 |
| Katalysatorvorstufe [mg/h] | 28,1 | 28,9 |
| Ligand [mg/h] | 156,5 | 160,7 |
| Umsatz [%] | 64 | 67 |
| Ausbeute [%] | 63 | 60 |
| Selektivität [%] | 97,3 | 89,8 |
| Raum-Zeit-Ausbeute [g/L·h] | 144 | 131 |

**Tabelle 5: Bedingungen und Ergebnisse der kontinuierlichen Herstellung von Vinylestern im Strömungsrohr mit Vinylacetat als Transvinylierungsreagenz bei erhöhtem Druck ([Ru(CO)₂OAc]ₙ)**

| Beispiel Nr. | 19 | 20 |
|---|---|---|
| Carbonsäure R-C(O)OH | Iso-Nonansäure (3,5,5-Trimethylhexansäure) | 2-Ethylhexansäure |
| Verweilzeit [min] | 75 | 75 |
| Reaktorvolumen [ml] | 100 | 100 |
| Temperatur [°C] | 140 | 140 |
| Druck [MPa] | 2 | 2 |
| Molares Verhältnis Carbonsäure:Vinylacetat: Katalysatorvorstufe | 1,0:5,0:0,0010 | 1,0:5,0:0,0010 |
| Katalysatorvorstufe | [Ru(CO)₂OAc]ₙ | [Ru(CO)₂OAc]ₙ, |
| Carbonsäure [g/h] | 19,8 | 18,5 |
| Vinylacetat [g/h] | 53,9 | 55,4 |
| Katalysatorvorstufe [mg/h] | 27,1 | 27,8 |
| Ligand [mg/h] | - | - |
| Umsatz [%] | 28 | 38 |
| Ausbeute [%] | 27 | 32 |
| Selektivität [%] | 94,8 | 84,7 |
| Raum-Zeit-Ausbeute [g/L·h] | 61 | 70 |

**Tabelle 6: Bedingungen und Ergebnisse der kontinuierlichen Herstellung von Vinylestern im Strömungsrohr mit Vinylacetat als Transvinylierungsreagenz und Verwendung der Einsatzcarbonsäure im Überschuss bei erhöhtem Druck (Pd(OAc)₂/1,10-Phenanthrolin)**

| Beispiel Nr. | 21 | 22 |
|---|---|---|
| Carbonsäure R-C(O)-OH | iso-Nonansäure (3,5,5-Trimethylhexansäure) | 2-Ethylhexansäure |
| Verweilzeit [min] | 75 | 75 |
| Reaktorvolumen [ml] | 100 | 100 |
| Temperatur [°C] | 140 | 140 |
| Druck [MPa] | 2 | 2 |
| Molares Verhältnis Carbonsäure:Vinylacetat: Katalysatorvorstufe | 1,0:0,2:0,001 | 1,0:0,2:0,001 |
| Molares Verhältnis Katalysatorvorstufe:Ligand | 1:8 | 1:8 |
| Katalysatorvorstufe | Pd(OAc)₂ | Pd(OAc)₂ |
| Ligand | 1,10-Phenanthrolin | 1,10-Phenanthrolin |
| Carbonsäure [g/h] | 65,1 | 65,0 |
| Vinylacetat [g/h] | 7,1 | 7,8 |
| Katalysatorvorstufe [g/h] | 0,0924 | 0,1012 |
| Ligand [g/h] | 0,5935 | 0,6497 |
| [a] Umsatz [%]^{[a]} | 19 | 18 |
| Ausbeute [%]^{[a]} | 18 | 17 |
| Selektivität [%] | 96,3 | 94,4 |
| Raum-Zeit-Ausbeute [g/L·h] | 139 | 141 |

| | | |
|---|---|---|
| ^{[a]} bezogen auf Einsatz an Carbonsäure R-C(O)-OH | | |

**Tabelle 7: Bedingungen und Ergebnisse der kontinuierlichen Herstellung von Vinylestern im Strömungsrohr mit Vinylacetat als Transvinylierungsreagenz und Anwendung von Verweilzeiten unter 1 h bei erhöhtem Druck (Pd(OAc)₂/1,10-Phenanthrolin)**

| Beispiel Nr. | 23 | 24 |
|---|---|---|
| Carbonsäure R-C(O)-OH | iso-Nonansäure (3,5,5-Trimethylhexansäure) | iso-Nonansäure (3,5,5-Trimethylhexansäure) |
| Verweilzeit [min] | 11 | 11 |
| Reaktorvolumen [ml] | 200 | 200 |
| Temperatur [°C] | 150 | 140 |
| Druck [MPa] | 2 | 2 |
| Molares Verhältnis Carbonsäure:Vinylacetat: Katalysatorvorstufe | 1,00:0,33:0,00165 | 1,00:5,00:0,002 |
| Molares Verhältnis Katalysatorvorstufe:Ligand | 1:5 | 1:8 |
| Katalysatorvorstufe | Pd(OAc)₂ | Pd(OAc)₂ |
| Ligand | 1,10-Phenanthrolin | 1,10-Phenanthrolin |
| Carbonsäure [g/h] | 838,2 | 263,7 |
| Vinylacetat [g/h] | 150,5 | 717,4 |
| Katalysatorvorstufe [g/h] | 1,9621 | 0,7483 |
| Ligand [g/h] | 7,8752 | 4,8051 |
| Umsatz [%]^{[a]} | 25 | 75 |
| Ausbeute [%]^{[a]} | 24 | 74 |
| Selektivität [%] | 97,8 | 98,5 |
| Raum-Zeit-Ausbeute [g/L·h] | 1193 | 1134 |

| | | |
|---|---|---|
| ^{[a]} bezogen auf Einsatz an Carbonsäure R-C(O)-OH | | |

Wie die Ergebnisse der Tabellen 1, 2, 4 und 5 zeigen, erzielt man bei der im stationären Zustand kontinuierlich betriebenen Transvinylierungsreaktion sehr hohe Raum-Zeit-Ausbeuten, die sich bei den bekannten Verfahren der Reaktivdestillation unter kontinuierlicher Entfernung von Vinylacetat und Essigsäure aus dem Reaktionsgeschehen nicht erzielen lassen. Ferner benötigen die bekannten Verfahren einen hohen Vinylacetateinsatz, da bei der Reaktivdestillation eine große Menge an Vinylacetat eingesetzt werden muss und ein großes Reaktionsvolumen belegt wird.

Ebenfalls kann, wie die in Tabelle 3 zusammengestellten Ergebnisse zeigen, auch bei einer Reaktionsführung unter Normaldruck eine hohe Raum-Zeit-Ausbeute erzielt werden. Auch bei einer Reaktionsführung, bei der Vinylacetat im molaren Unterschuss verwendet wird, können hohe Raum-Zeit-Ausbeuten an dem gewünschten Vinylester erhalten werden (Tabelle 6). Gemäß Tabelle 7 können die Verweilzeiten im Strömungsrohr auf unter 1 Stunde eingestellt werden, so dass sich Betriebszustände mit hoher Belastung einstellen lassen, die eine deutliche Steigerung der Raum-Zeit-Ausbeute ermöglichen.

Das im Entspannungsgefäß (7) erhaltene Rohprodukt wurde über Leitung (9) auf einen Dünnschichtverdampfer (10) gegeben, aus dem bei einer Manteltemperatur von 95°C und im Unterdruck das Kopfprodukt enthaltend Vinylacetat oder Vinylpropionat, Essigsäure oder Propionsäure und den jeweiligen Vinylester R-C(O)O-CH = CH₂ abgezogen wurde. Dieser Produktstrom wurde mit den gasförmigen Anteilen aus dem Entspannungsgefäß (7) vereinigt und über Leitung (12) auf die Destillationskolonne (13) geführt, in der das Produktgemisch in eine Kopffraktion aus Vinylacetat oder Vinylpropionat und in ein Sumpfprodukt aus Essigsäure oder Propionsäure und dem jeweiligen Vinylester R-C(O)O-CH = CH₂ aufgespalten wurde. Der vinylacetat- oder vinylpropionathaltige Strom wurde über Leitung (14) zurückgeführt und das Sumpfprodukt über Leitung (15) auf eine weitere Destillationskolonne (16) gegeben, in der als Kopfprodukt Essigsäure oder Propionsäure abgezogen wurde. Als Sumpfprodukt wurde über Leitung (18) der jeweilige Vinylester R-C(O)O-CH = CH₂ gewonnen. Die jeweiligen Destillationsbedingungen an dem Dünnschichtverdampfer (10) und an den Destillationskolonnen (13) und (16) ließen sich durch routinemäßiges Optimieren einstellen.

Aus dem flüssigen Austrag des Dünnschichtverdampfers (10) wurden, bezogen auf 100 Massenteile, etwa 5-10 Massenteile eines hochsiederhaltigen Seitenstroms über Leitung (20) ausgeschleust während der Rest als Katalysatorkreislauf zurückgeführt wurde. Über Leitung (22) wurde Frischkatalysator ergänzt, entsprechend den im Strömungsrohr (5) einzustellenden Verhältnissen. Die Ergänzung des Frischkatalysators erfolgte in Lösung, indem Palladiumacetat und 1,10-Phenanthrolin oder 2,2'-Bipyridyl oder [Ru(CO)₂OAc]ₙ in einer Mischung aus Vinylacetat oder Vinylpropionat und der jeweiligen Einsatzcarbonsäure eingetragen wurden.

## Patentansprüche

1. Kontinuierliches, katalytisches Verfahren zur Herstellung eines Vinylesters der Formel R-C(O)O-CH = CH₂ durch Umsetzung einer Carbonsäure der Formel R-C(O)OH mit einem Transvinylierungsreagenz der Formel R¹-C(O)O-CH = CH₂, wobei R und R¹ unabhängig voneinander einen aliphatischen, cycloaliphatischen oder aromatischen Rest bedeuten, **dadurch gekennzeichnet, dass** die Umsetzung bei einer Temperatur von 90 bis 160°C und bei einem Druck von 0,8 bis 8 MPa ohne Entzug eines Reaktionspartners in Gegenwart eines Übergangsmetallkatalysators enthaltend mindestens ein Übergangsmetall aus der Gruppe von Ruthenium, Osmium, Rhodium, Iridium, Palladium und Platin erfolgt und anschließend das erhaltene Reaktionsgemisch in seine Bestandteile aufgetrennt wird.

2. Verfahren gemäß Anspruch 1, **dadurch gekennzeichnet, dass** die Umsetzung bei einer Temperatur von 90 bis 150°C und insbesondere von 90 bis 140°C durchgeführt wird.

3. Verfahren gemäß Anspruch 1 oder 2, **dadurch gekennzeichnet, dass** die Umsetzung bei einem Druck von 0,8 bis 2 MPa erfolgt.

4. Verfahren gemäß Anspruch 3, **dadurch gekennzeichnet, dass** die Umsetzung bei einem Druck von 0,8 bis 2 MPa und bei einer Temperatur von 90 bis 140°C durchgeführt wird.

5. Verfahren gemäß einem oder mehreren der Ansprüche 1 bis 4, **dadurch gekennzeichnet, dass** in der Carbonsäure der Formel R-C(O)OH der Rest R 2 bis 20 Kohlenstoffatome enthält.

6. Verfahren gemäß Anspruch 5, **dadurch gekennzeichnet, dass** die Carbonsäure der Formel R-C(O)OH ausgewählt wird aus der Gruppe von Propionsäure, n-Buttersäure, iso-Buttersäure, n-Valeriansäure, 2-Methylbuttersäure, 3-Methylbuttersäure, Pivalinsäure, n-Heptansäure, 2-Methylhexansäure, 2-Ethylhexansäure, n-Octansäure, n-Nonansäure, Isononansäure, Neononansäure, 3,5,5-Trimethylhexansäure, n-Decansäure, 2-Propylheptansäure, Neodecansäure, einem Gemisch isomerer C9- bis C11-Säuren, einem Gemisch isomerer C15- bis C19-Säuren, Laurinsäure, Tridecansäure, Palmitinsäure, Stearinsäure, Benzoesäure, Naphthalincarbonsäure, Acrylsäure, Crotonsäure und Methacrylsäure.

7. Verfahren gemäß einem oder mehreren der Ansprüche 1 bis 4, **dadurch gekennzeichnet, dass** in dem Transvinylierungsreagenz der Formel R¹-C(O)O-CH = CH₂ der Rest R¹1 bis 20 Kohlenstoffatome enthält.

8. Verfahren gemäß Anspruch 7, **dadurch gekennzeichnet, dass** R¹ für Methyl, Ethyl oder Undecyl steht.

9. Verfahren gemäß einem oder mehreren der Ansprüche 1 bis 8, **dadurch gekennzeichnet, dass** der Übergangsmetallkatalysator ein- oder mehrzähnige Organostickstoff- oder Organophosphorliganden komplex gebunden enthält.

10. Verfahren gemäß einem oder mehreren der Ansprüche 1 bis 9, **dadurch gekennzeichnet, dass** die Gesamtkonzentration des Übergangsmetalls oder der Übergangsmetalle 0,005 bis 1,5 mol-%, vorzugsweise von 0,01 bis 1,0 mol-% und insbesondere von 0,02 bis 0,6 mol-% beträgt, jeweils bezogen auf die im Unterschuss eingesetzte Ausgangsverbindung.

11. Verfahren gemäß einem oder mehreren der Ansprüche 1 bis 10, **dadurch gekennzeichnet, dass** das molare Verhältnis von Übergangsmetall zu ein- oder mehrzähnigem Organostickstoff- oder Organophosphorligand von 1:1 bis 1:40, vorzugsweise von 1:3 bis 1:30 beträgt.

12. Verfahren gemäß einem oder mehreren der Ansprüche 1 bis 11, **dadurch gekennzeichnet, dass** man als Übergangsmetall Palladium und als mehrzähnigen Organostickstoffligand 1,10-Phenanthrolin oder 2,2'-Bipyridyl verwendet.

13. Verfahren gemäß einem oder mehreren der Ansprüche 1 bis 8, **dadurch gekennzeichnet, dass** der Übergangsmetallkatalysator zusätzlich ein redoxaktives Übergangsmetall der Gruppe Ib des Periodensystems der Elemente und eine Alkalimetallverbindung enthält.

14. Verfahren gemäß Anspruch 13, **dadurch gekennzeichnet, dass** man neben dem Übergangsmetall zusätzlich als redoxaktives Übergangsmetall der Gruppe Ib des Periodensystems der Elemente Kupfer verwendet und als Alkalimetallverbindung eine Lithiumverbindung ausgewählt aus der Gruppe von Lithiumcarboxylaten, Lithiumcarbonat, Lithiumhydrogencarbonat, Lithiumchlorid und Lithiumhydroxid.

15. Verfahren gemäß Anspruch 14, **dadurch gekennzeichnet, dass** man als Übergangsmetall Palladium verwendet.

16. Verfahren gemäß einem oder mehreren der Ansprüche 1 bis 15, **dadurch gekennzeichnet, dass** die Umsetzung in einem Rohrreaktor erfolgt.

17. Verfahren gemäß Anspruch 16, **dadurch gekennzeichnet, dass** der Rohrreaktor mit einem Umpump und gegebenenfalls mit einem Wärmetauscher versehen ist.

## Claims

1. Continuous, catalytic process for preparing a vinyl ester of the formula R-C(O)O-CH=CH₂ by reaction of a carboxylic acid of the formula R-C(O)OH with a transvinylating reagent of the formula R¹-C(O)O-CH=CH₂ where R and R¹ are each independently an aliphatic, cycloaliphatic or aromatic radical, **characterized in that**
the reaction is effected at a temperature of 90 to 160°C and at a pressure of 0.8 to 8 MPa without withdrawal of a reactant in the presence of a transition metal catalyst containing at least one transition metal from the group of ruthenium, osmium, rhodium, iridium, palladium and platinum, and then the resulting reaction mixture is separated into its constituents.

2. Process according to Claim 1, **characterized in that** the reaction is conducted at a temperature of 90 to 150°C and especially of 90 to 140°C.

3. Process according to Claim 1 or 2, **characterized in that** the reaction is effected at a pressure of 0.8 to 2 MPa.

4. Process according to Claim 3, **characterized in that** the reaction is conducted at a pressure of 0.8 to 2 MPa and at a temperature of 90 to 140°C.

5. Process according to one or more of Claims 1 to 4, **characterized in that** the R radical in the carboxylic acid of the formula R-C(O)OH contains 2 to 20 carbon atoms.

6. Process according to Claim 5, **characterized in that** the carboxylic acid of the formula R-C(O)OH is selected from the group of propionic acid, n-butyric acid, isobutyric acid, n-valeric acid, 2-methylbutyric acid, 3-methylbutyric acid, pivalic acid, n-heptanoic acid, 2-methylhexanoic acid, 2-ethylhexanoic acid, n-octanoic acid, n-nonanoic acid, isononanoic acid, neononanoic acid, 3,5,5-trimethylhexanoic acid, n-decanoic acid, 2-propylheptanoic acid, neodecanoic acid, a mixture of isomeric C9 to C11 acids, a mixture of isomeric C 15 to C 19 acids, lauric acid, tridecanoic acid, palmitic acid, stearic acid, benzoic acid, naphthoic acid, acrylic acid, crotonic acid and methacrylic acid.

7. Process according to one or more of Claims 1 to 4, **characterized in that** the R¹ radical in the transvinylating reagent of the formula R¹-C(O)O-CH=CH₂ contains 1 to 20 carbon atoms.

8. Process according to Claim 7, **characterized in that** R¹ is methyl, ethyl or undecyl.

9. Process according to one or more of Claims 1 to 8, **characterized in that** the transition metal catalyst contains mono- or polydentate organonitrogen or organophosphorus ligands in complexed form.

10. Process according to one or more of Claims 1 to 9, **characterized in that** the total concentration of the transition metal(s) is 0.005 to 1.5 mol%, preferably from 0.01 to 1.0 mol% and especially from 0.02 to 0.6 mol%, based in each case on the starting compound used in deficiency.

11. Process according to one or more of Claims 1 to 10, **characterized in that** the molar ratio of transition metal to mono- or polydentate organonitrogen or organophosphorus ligands is from 1:1 to 1:40, preferably from 1:3 to 1:30.

12. Process according to one or more of Claims 1 to 11, **characterized in that** the transition metal used is palladium and the polydentate organonitrogen ligand used is 1,10-phenanthroline or 2,2'-bipyridyl.

13. Process according to one or more of Claims 1 to 8, **characterized in that** the transition metal catalyst additionally contains a redox-active transition metal from group Ib of the periodic table of the elements and an alkali metal compound.

14. Process according to Claim 13, **characterized in that**, as well as the transition metal, copper is additionally used as a redox-active transition metal from group Ib of the periodic table of the elements, and a lithium compound selected from the group of lithium carboxylates, lithium carbonate, lithium hydrogencarbonate, lithium chloride and lithium hydroxide is used as an alkali metal compound.

15. Process according to Claim 14, **characterized in that** the transition metal used is palladium.

16. Process according to one or more of Claims 1 to 15, **characterized in that** the reaction is effected in a tubular reactor.

17. Process according to Claim 16, **characterized in that** the tubular reactor is provided with a circulation pump and optionally with a heat exchanger.

## Revendications

1. Procédé catalytique continu pour la fabrication d'un ester vinylique de formule R-C(O)O-CH=CH₂ par mise en réaction d'un acide carboxylique de formule R-C(O)OH avec un réactif de transvinylation de formule R¹-C(O)O-CH=CH₂, R et R¹ signifiant indépendamment l'un de l'autre un radical aliphatique, cycloaliphatique ou aromatique, **caractérisé en ce que** la réaction a lieu à une température de 90 à 160 °C et à une pression de 0,8 à 8 MPa sans extraction d'un partenaire de réaction en présence d'un catalyseur de métal de transition contenant au moins un métal de transition du groupe constitué par le ruthénium, l'osmium, le rhodium, l'iridium, le palladium et le platine, puis le mélange réactionnel obtenu est séparé en ses constituants.

2. Procédé selon la revendication 1, **caractérisé en ce que** la réaction est réalisée à une température de 90 à 150 °C et notamment de 90 à 140 °C.

3. Procédé selon la revendication 1 ou 2, **caractérisé en ce que** la réaction a lieu à une pression de 0,8 à 2 MPa.

4. Procédé selon la revendication 3, **caractérisé en ce que** la réaction est réalisée à une pression de 0,8 à 2 MPa et à une température de 90 à 140 °C.

5. Procédé selon une ou plusieurs des revendications 1 à 4, **caractérisé en ce que** le radical R dans l'acide carboxylique de formule R-C(O)OH contient 2 à 20 atomes de carbone.

6. Procédé selon la revendication 5, **caractérisé en ce que** l'acide carboxylique de formule R-C(O)OH est choisi dans le groupe constitué par l'acide propionique, l'acide n-butyrique, l'acide isobutyrique, l'acide n-valérique, l'acide 2-méthylbutyrique, l'acide 3-méthylbutyrique, l'acide pivalique, l'acide n-heptanoïque, l'acide 2-méthylhexanoïque, l'acide 2-éthylhexanoïqe, l'acide n-octanoïque, l'acide n-nonanoïque, l'acide isononanoïque, l'acide néononanoïque, l'acide 3,5,5-triméthylhexanoïque, l'acide n-décanoïque, l'acide 2-propylheptanoïque, l'acide néodécanoïque, un mélange d'acides en C9 à C11 isomères, un mélange d'acides en C15 à C19 isomères, l'acide laurique, l'acide tridécanoïque, l'acide palmitique, l'acide stéarique, l'acide benzoïque, l'acide naphtoïque, l'acide acrylique, l'acide crotonique et l'acide méthacrylique.

7. Procédé selon une ou plusieurs des revendications 1 à 4, **caractérisé en ce que** le radical R¹ dans le réactif de transvinylation de formule R¹-C(O)O-CH=CH₂ contient 1 à 20 atomes de carbone.

8. Procédé selon la revendication 7, **caractérisé en ce que** R¹ représente méthyle, éthyle ou undécyle.

9. Procédé selon une ou plusieurs des revendications 1 à 8, **caractérisé en ce que** le catalyseur de métal de transition contient des ligands organo-azote ou organophosphore mono- ou polydentates reliés sous forme complexée.

10. Procédé selon une ou plusieurs des revendications 1 à 9, **caractérisé en ce que** la concentration totale du métal de transition ou des métaux de transition est de 0,005 à 1,5 % en moles, de préférence de 0,01 à 1,0 % en moles et notamment de 0,02 à 0,6 % en moles, à chaque fois par rapport au composé de départ utilisé en déficit.

11. Procédé selon une ou plusieurs des revendications 1 à 10, **caractérisé en ce que** le rapport molaire entre le métal de transition et le ligand organo-azote ou organophosphore mono- ou polydentate est de 1:1 à 1:40, de préférence de 1:3 à 1:30.

12. Procédé selon une ou plusieurs des revendications 1 à 11, **caractérisé en ce que** du palladium est utilisé en tant que métal de transition et de la 1,10-phénanthroline ou du 2,2'-bipyridyle en tant que ligand organo-azote polydentate.

13. Procédé selon une ou plusieurs des revendications 1 à 8, **caractérisé en ce que** le catalyseur de métal de transition contient en outre un métal de transition à activité redox du groupe Ib du tableau périodique des éléments et un composé de métal alcalin.

14. Procédé selon la revendication 13, **caractérisé en ce qu'**en plus du métal de transition, du cuivre est en outre utilisé en tant que métal de transition à activité redox du groupe Ib du tableau périodique des éléments et un composé de lithium choisi dans le groupe constitué par les carboxylates de lithium, le carbonate de lithium, l'hydrogénocarbonate de lithium, le chlorure de lithium et l'hydroxyde de lithium en tant que composé de métal alcalin.

15. Procédé selon la revendication 14, **caractérisé en ce que** du palladium est utilisé en tant que métal de transition.

16. Procédé selon une ou plusieurs des revendications 1 à 15, **caractérisé en ce que** la réaction a lieu dans un réacteur tubulaire.

17. Procédé selon la revendication 16, **caractérisé en ce que** le réacteur tubulaire est muni d'une pompe de circulation et éventuellement d'un échangeur de chaleur.
